(19) Europäisches Patentamt
European Patent Office
Office européen des brevets

(11) Veröffentlichungsnummer: **0 000 903**
**B1**

(12) **EUROPÄISCHE PATENTSCHRIFT**

(45) Veröffentlichungstag der Patentschrift: **20.01.82**

(51) Int. Cl.³: **B 41 M 5/00**, B 01 J 13/02 //C08G18/78, C07D273/04

(21) Anmeldenummer: **78100634.1**

(22) Anmeldetag: **09.08.78**

(54) **Reaktionsdurchschreibepapiere und deren Herstellung.**

(30) Priorität: **26.08.77 DE 2738509**

(43) Veröffentlichungstag der Anmeldung:
**07.03.79 Patentblatt 79/5**

(45) Bekanntmachung des Hinweises auf die Patenterteilung:
**20.01.82 Patentblatt 82/3**

(84) Benannte Vertragsstaaten:
**BE CH DE FR GB NL SE**

(56) Entgegenhaltungen:
DE - A - 1 519 853
DE - A - 2 221 756
DE - A - 2 242 910
DE - A - 2 251 381
US - A - 3 432 327
US - A - 3 748 329

(73) Patentinhaber: **BAYER AG**
**Zentralbereich Patente, Marken und Lizenzen**
**D-5090 Leverkusen 1, Bayerwerk (DE)**

(72) Erfinder: **Baatz, Günther, Dr.**
**Zimmermannstrasse 2**
**D-8941 Buxheim (DE)**
Erfinder: **Schäfer, Walter, Dr.**
**Wolfskaul 6**
**D-5000 Köln 80 (DE)**
Erfinder: **Findeisen, Kurt, Dr.**
**In der Follmühle 10**
**D-5068 Odenthal 2 (DE)**
Erfinder: **Dahm, Manfred, Dr.**
**Am Falkenberg 29**
**D-5090 Leverkusen (DE)**

**EP 0 000 903 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Reaktionsdurchschreibepapiere und deren Herstellung

Reaktionsdurchschreibepapiere sind bekannt (vergl. M. Gutcho, Capsule Technology and Microencapsulation, Noyes Data Corporation 1972, Seiten 242—277; G. Baxter in Microencapsulation, Processes and Applications, herausgegeben von J. E. Vandegaer, Plenum Press, New York, London, Seiten 127—143).

Reaktionsdurchschreibepapiere sind auch aus der US—PS 3.432.327 bekannt. Man versteht darunter Papiere, in denen zwei Schichten miteinander in Kontakt stehen, von denen jede eine Farbbildungskomponente enthält, die mit der jeweils anderen unter Bildung eines Farbstoffes reagiert. Mindestens eine der Farbbildungskomponenten liegt in Form von Mikrokapseln vor, die durch den Druck eines Schreibwerkzeuges zerbrechen und die eingekapselte Komponente zur Reaktion mit der zweiten freigeben. Die beiden Farbbildungsschichten können übereinander auf einer Papieroberfläche oder getrennt auf zwei miteinander in Kontakt stehenden Papieroberflächen aufgetragen sein.

Für die Herstellung der Mikrokapseln benutzt man gemäß US—PS 3.432.327 die Phasengrenzflächenpolymerisation, eine Polyreaktion an der Phasengrenzfläche einer hydrophilen und einer hydrophoben Flüssigkeit. Die Reaktion zum Farbstoff muß nach der US-Patentschrift im hydrophilen Medium ablaufen. Deshalb wird zunächst eine Farbbildungskomponente in einer hydrophilen Flüssigkeit, beispielsweise Wasser, gelöst und dann dieser Lösung die erste Komponente des Kapselwandmaterials zugegeben. Die zweite Komponente des Kapselwandmaterials wird in einer hydrophoben Flüssigkeit (Öl, Paraffin, aromatisches Lösungsmittel) gelöst, und dann die hydrophile Lösung in der hydrophoben dispergiert. An den Phasengrenzflächen der dispergierten hydrophilen Tröpfchen bildet sich dann aus den beiden Kapselwandkomponenten die Mikrokapsel.

Die zweite Farbbildungskomponente kann ebenfalls einegekapselt werden; in der Regel ist sie allerdings nicht eingekapselt.

Paare von geeigneten Farbbildungskomponenten sind in der US-Patentschrift in großer Zahl angegeben; es kann sich u. a. um anorganische Salze (z.B. Kaliumhexacyanoferrat II - Ammonium-Eisen-(III)-Sulfat), gefärbte Metallkomplexe (z.B. Diacetyl-dioxim-Nickelacetat) und organische Farbstoffe (z.B. Bromkresol-purpur-Natriumhydroxid) haldeln.

Kapselwandbildungskomponenten sind ebenfalls in großer Zahl angegeben, beispielsweise die Kombination bestimmter ausgewählter Diisocyanate und Wasser, Diol oder Diamin.

Die Arbeitsweise der US-Patentschrift 3.432.327 ist darauf gerichtet, eine Lösung eines Farbbildners in einem hydrophilen Lösungsmittel, bevorzugt Wasser, einezukapseln.

Dieses ist aber von entscheidendem Nachteil, denn für Wasser undurchlässige Kapselfilme sind bisher praktisch nicht herstellbar. Somit sind diese Kapseln nicht lagerstabil, da sie nach mehr oder weniger kurzer Zeit austrocknen. Daraus hergestellte Durchschreibepapiere verlieren somit ihre Durchschreibefähigkeit rasch.

Aus der Deutschen Offenlegungsschrift 23 11 712 ist weiterhin die Verwendung von Umsetzungsprodukten aus Di- oder Polyolen vom Molekulargewicht 400—10.000 und Di- oder Polyisocyanaten als Isocyanatkomponenten bei der Mikroverkapselung bekannt. Man kann so Lösungen von Farbstoffvorstufen für Durchschreibepapiere einkapseln; diese Kapseln sind für die für das Verfahren notwendigen, meist aromatischen und alkylaromatischen Lösungsmittel nicht undurchlässig, obwohl dies für die Wirkung der Reaktionsdurchschreibepapiere unbedingt erforderlich ist. Sie neigen uch sehr stark zur Agglomeration. Kapselagglomerate stören erheblich, weil einzelne Kapseln schon bei der Herstellung der Papiere zerstört werden und so ein fleckiges Papier erhalten wird. Unter ungünstigen Bedingungen wird sogar die Schreibleistung der Papiere bedeutend herabgesetzt. Man braucht daher einzelne, nicht zur Agglomeration neigende Kapseln für Durchschreibepapiere.

Aus der DE—OS 22 51 381 schließlich ist bekannt, Farbbildner in Kapseln einzuschließen, deren Wände aus Polyharnstoff bestehen. Auf Seite 12 wird mitgeteilt, daß gute Ergebnisse nicht immer erhalten werden, sondern insbesondere dann, wenn man Polyisocyanat-Addukte oder Polyamin-Addukte benutzt. In den konkret angegebenen Fällen ist jedoch die Dichtigkeit und die Alterungsbeständigkeit der Mikrokapseln unzureichend.

Die Mikrokapseln für die Herstellung von Durchschreibepapieren

(1.) müssen für den Farbbildner und sein Lösungsmittel undurchlässig sein. Durchlässigkeit für den Farbbildner führt zur Verfärbung, Durchlässigkeit für das Lösungsmittel zum Eintrocknen des Kapselinhalts und damit zur Wirkungslosigkeit;

(2.) dürfen erst unter dem Druck des Schreibgerätes zerbrechen. Die Kapselwand muß also andersartige Belastungen überstehen;

(3.) sollen möglichst als individuelle Teilchen vorliegen und nicht als größere Agglomerate;

(4.) müssen sich leicht auf die Papieroberfläche aufbringen und sofort fixieren lassen. Dazu müssen sie so temperaturstabil sein, daß sie Temperaturen bis zu 100°C beim Trocknen unbeschadet überstehen.

Weiterhin ist es günstig, möglichst wenig Hüllmaterial zu verwenden, und die Kapselwände möglichst dicht gegenüber äußeren Einflüssen, insbesondere gegen Sauerstoff, Lichteinwirkung und Säuren oder Basen zu machen.

Der Erfindung liegt die Erkenntnis zugrunde, daß Wände von Farbbildnerlösungen enthaltenden Mikrokapseln für Reaktionsdurchschreibepapiere erhalten werden aus, einerseits, Diisocyanaten der Formel (I)

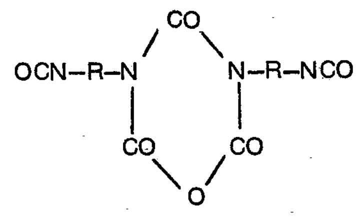
(I)

worin R einen Alkylenrest mit 2 bis 10 C-Atomen bedeutet und, andererseits, einem Diamin. Die aus dem Reaktionsprodukt resultierenden Kapselmembranen erfüllen in hervorragender Weise die oben angegebenen Anforderungen, wenn hydrophobe organische Lösungen der Farbbildner eingekapselt werden.

Gegenstand der Erfindung ist ein Reaktionsdurchschreibepapier, das eine Lösung eines Farbbildners in mikroverkapselter Form enthält, wobei der Farbbildner als Lösung in einem hydrophoben organischen Lösungsmittel eingekapselt ist, dadurch gekennzeichnet, dass die Kapselwände aus dem Polyadditionsprodukt eines Diisocyanats der Formel (I)

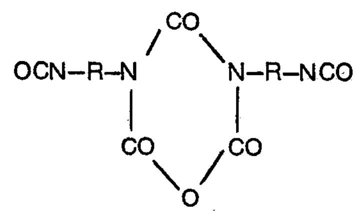
(I)

worin R einen Alkylenrest mit 2 bis 10 C-Atomen bedeutet, und einem Diamin.

Ein weiterer Gegenstand der Erfindung ist die Verwendung einer Lösung eines Farbbildners in einem hydrophoben, organischen Lösungsmittel als Kernmaterial in Mikrokapseln, deren Wände aus dem Polyadditionsprodukt eines Diisocyanats der Formel (I) und einem Diamin bestehen, zur Herstellung von Reaktionsdurchschreibepapieren.

Neben den schon genannten ergibt sich ein wieterer Vorteil bei Verwendung dieser Isocyanate durch die hervorragende Löslichkeit in vielen Lösungsmitteln, die für die Lösung der Farbstoffvorstufen infrage kommen. Dadurch erhält man einen Freiheitsgrad in der Auswahl der Lösungsmittel, der besonders im Hinblick auf die Umweltbelastung von Bedeutung ist. Man kann so durch Kombination das für die Lösung des Farbstoffes am besten geeignete und gleichzeitig das die Umwelt am wenigsten belastende Lösungsmittel optimal auswählen.

Die Diisocyanate der Formel (I) stellen Derivate des 2,4,6-Triketo-1,3,5-Oxadiazins mit zwei freien Isocyanat-Gruppen dar. Besonders geeignet sind die Verbindungen, in denen die Reste R vom Butan, Hexan und Octan abstammen. Besonders bevorzugt ist der n-Hexanrest. Die Produkte und ihre Herstellung sind aus der DE—AS 1.670.666 bekannt.

Geeignete Diamine sind aliphatische primäre oder sekundäre Diamine wie z.B.:

Äthylendiamin-(1,2), Bis(3-aminopropyl)-amin, Hydrazin, Hydrazinäthanol-(2), Bis-(2-methylaminoäthyl)-methylamin, 1,4-Diaminobenzol, 4,4'-Diaminodiphenyl-methan, 1,4-Diaminocyclohexan, 3-Amino-1-methyl-aminopropan, N-Hydroxyäthyläthylendiamin, N-Methyl-bis(3-aminopropyl)-amin, 1,4-Diamino-n-butan, 1,6-Diamino-n-hexan, Äthylen-(1,2)-diamin-N-äthan-sulfonsäure (als Alkalisalz), 1-Aminoäthyläthylendiamin-(1,2), Bis-(N,N'-aminoäthyl)-äthylendiamin-(1,2).

Farbbildner sind im wesentlichen farblose, basische Produkte, die verschiedene chromophore Gruppen haben. Beispiele hierfür sind Bis-(p-aminoaryl)-phthalide, Leukoauramine, Acylauramine, $\alpha,\beta$-ungesättigte Arylketone, basische Monoazofarbstoffe, Rhodamin-B-Lactame wie die N-(p-nitrophenyl)-Rhodamin B-Lactame, durch Aminogruppen substituierte Polyarylcarbinole und deren Umsetzungsprodukte, z.B. deren Ester oder Äther und verschiedene heterocyclische Spirane. Bevorzugte Verbindungen sind 3,3-Bis-(p-dimethylaminophenyl)-6-dimethylamino-phthalid (Kristallviolettlacton). Benzoylleukomethylenblau und Derivate von Michlers Hydrol, insbesondere das p-Toluolsulfinat von Michlers Hydrol.

Lösungsmittel für die basische Farbbildnerkomponente (Farbstoffvorstufe) und für die Diisocyanate sind im allgemeinen aromatische Kohlenwasserstoffe, die auch durch Alkyl oder Halogen substituiert sein können.

Beispiele sind chlorierte Diphenyle, Dodecylbenzol, Gemische aus teilhydrierten und nichthydrierten Terphenylen, Isopropyldiphenyl, Diisopropylbenzol, Benzoesäureäthylester, Gemische aus Diphenyl und Diphenyläther, Phthalsäurebutylester, Aralkyl- oder Diaryläther, die Xylole od. handelsübliche, in den Aromatisierungsanlagen der Petrochemie und Erdölchemie anfallende Aromatengemische.

Lösungsmittel für die Diamine ist im allgemeinen Wasser.

Beim Beschreiben der Reaktionsdurchschreibepapiere trifft das auslaufende Kernmaterial auf die Nehmerschicht, die eine Beschichtung besitzt, auf der aus den farblosen Farbstoffvorstufen Farbstoffe entstehen, die nun die Durchschrift erscheinen lassen. Beschichtungsmaterialien sind dabei natürliche und synthetische Produkte, wie Kaolin, Attapulgit, Montmorillonit, Betonit, saure Bleicherde oder Phenolharze. Man kann z.B. in der Geberschicht, d.h. der Mikrokapselschicht, säure-aktivierbare Farbstoffe und in der Nehmerschicht sauer reagierende Komponenten einsetzen.

Die Herstellung der Mikrokapseln kann in verschiedener Weise erfolgen. So kann man zunächst das Diisocyanat und die Farbstoffvorstufe in einem geeigneten Lösungsmittel lösen und diese organische Phase in einer wäßrigen Diaminlösung, die gegebenenfalls auch Schutzkolloide enthalten kann, emulgieren. Es ist auch möglich, die organische Phase zunächst mit oder ohne Tenside und Schutzkolloide in Wasser bis zu einer gewünschten Teilchengröße zu emulgieren und der wäßrigen Phase erst dann das für die Kapselwandbildung notwendige Diamin zuzusetzen.

Zur Emulgierung und Stabilisierung der gebildeten Emulsion werden der wäßrigen Phase Emulgierhilfsmittel zugesetzt. Beispiele für solche, als Schutzkolloide wirkende Produkte sind Carboxymethylcellulose, Gelatine und Polyvinylalkohol. Beispiele für Emulgatoren sind oxäthyliertes 3-Benzylhydroxybiphenyl, Umsetzungsprodukte von Nonylphenol mit unterschiedlichen Mengen Äthylenoxid und Sorbitanfettsäureester.

Den Verlauf der kapselwandbildenden Polyaddition kann man anhand des Aminverbrauchs verfolgen. Nach vollständiger Umsetzung der freien Isocyanatgruppen kann man durch Temperaturerhöhung den Oxadiazin-Ring des Isocyanats öffnen, wobei sich eine neue Isocyanat-Gruppe bildet, die mit dem bereits benutzten oder einem anderen Amin unter Vernetzung und Härtung des Polyadditionsproduktes reagieren kann.

Die Mikrokapseln können kontinuierlich oder diskontinuierlich hergestellt werden. Man verwendet im allgemeinen Dispergiergeräte, die ein Schergefälle erzeugen. Beispiele hierfür sind Blatt-, Korb-, Schnellrührer, Kolloidmühlen, Homogenisatoren, Ultraschalldispergatoren, Düsen, Strahldüsen, Supratonmaschinen. Die Stärke der Turbulenz beim Vermischen ist in erster Linie bestimmend für den Durchmesser der erhaltenen Mikrokapseln. Kapseln in der Größe von 1 bis 2000 $\mu$m können hergestellt werden. Bevorzugt sind Kapseln mit Durchmessern von 2 bis 20 $\mu$m. Die Kapseln agglomerieren nicht und haben eine enge Teilchengrößenverteilung. Das Gewichtsverhältnis von Kernmaterial zu Hüllenmaterial ist 50—90 zu 50—10.

Die druckempfindlichen Durchschreibepapiere werden in bekannter Weise hergestellt (vergl. M. Gutcho, Capsule Technology and Microencapsulation, Noyes Data Corporation, 1972, Seiten 242—277). Die primär erhaltenen Mikrokapselsuspensionen enthalten im allgemeinen 10 bis 35 Gew.-% Kapseln. Sie niegen etwas zum Aufrahmen, solange sie kein Bindemittel enthalten. Dies läßt sich für eine Konzentration ausnutzen. Die bevorzugte Kapselgröße liegt um 10 $\mu$m. Die homogenisierten, mit Binder und gegebenenfalls inerten Füllstoffen wie Talkum oder Kaolin versehenen Kapselsuspensionen können manuell mit einem Blumendrahtrakel oder maschinell mit einer Luftbürste in Auftragsmengen von 4—8 g/m$^2$ auf Rohpapier (z.B. von 40 bis 100 g/m$^2$) aufgetragen werden. Die Beschichtung von Rohpapieren ist beschrieben in den Deutschen Offenlegungsschriften 1.934.457 und 1.955.542. Die so beschichteten Papiere enthalten die erste Farbbildungskomponente; sie werden als Geberkomponenten bezeichnet.

Die Geberkomponente ist im allgemeinen bei Durchschreibesätzen die Rückseite des obersten Blattes. Die Vorderseite des nächsten Blattes ist mit der zweiten Farbgeberkomponente beschichtet. Man bezeichnet diese Schicht als Nehmerkomponente. Bei Durchschreibesätzen ist die Nehmerkomponente die Oberseite des zweiten Papierblattes. Bei Mehrfachdurchschriebesätzen müssen die folgenden Geberblätter auf der Gegenseite eine Nehmerbeschichtung tragen. Die Herstellung solcher Nehmerschichten ist bekannt und ebenfalls in den Deutschen Offenlegungsschriften 1.934.457 und 1.955.542 beschrieben.

## Beispiel 1

(a) Herstellung des wendbildenden Materials

2000 g n-Hexan-1,6-diisocyanat werden in einem 2,7 l Autoklaven mit 1 g Tri-p-tolylarsinoxid versetzt und 8 Stunden bei einem $CO_2$-Druck von 3 atü bei 50°C gerührt.

Hierbei werden 17,5 l Kohlendioxid aufgenommen. Die Reaktion wird durch Zugabe von 2 g Phosphortrichlorid unterbrochen und das Reaktionsprodukt von nicht umgesetztem Ausgangsprodukt im Dünnschichverdampfer abgetrennt (2-maliger Durchgang, Heiztemperatur 180°C bei 1 Torr).

Man erhält 1957 g Oxadiazinon mit einem NCO-Wert von 19,0% und einer Viskosität von 2480 cp/50°C.

Das IR-Spektrum zeigt die charakteristischen Carbonyl-Absorptionsbanden bei 5,5 bis 5,71 und 5,82 $\mu$m.

(b) Verkapselung

In 25 g Solventnaphtha (Aromatengemisch der BV Aral aus Xylol, Cumol, Tuluol und weiteren Naphthenölen) werden unter Rühren und Erwärmen auf 70°C 0,75 g Kristallviolettlacton und 0,25 g N-Benzoyl-leukomethylenblau gelöst.

Nach dem Abkühlen der Lösung werden 5 g des unter Beispiel 1 (a) beschriebenen Oxadiazinons zugegeben und gelöst.

Die homogene organische Phase wird anschließend in 300 g Wasser emulgiert, welches 1,5 g Mowiol 56—98 (Polyvinylalkohol der Hoechst AG) als Emulgierhilfsmittel enthält.

Zum Emulgieren wird eine Kotthoff-Mischsirene verwendet (8900 U/Min., 1 l Becherglas).

Nach ca. 1 Min. Emulgierzeit wird die Mischsirene durch einen Laborrührer vom Typ Lenart-Rapid ersetzt (500 U/Min.). Gleichzeitig wird dem Ansatz eine Lösung von 0,7 g 1-Aminoäthyläthylen-diamin-1,2-(Diäthylentriamin) in 70 g Wasser zugestezt. Unter stetigem Rühren wird der Ansatz schnell auf 70°C erwärmt und ca. 1 Std. auf dieser Temperatur gehalten. Die Heizphase dient lediglich der Ausbildung einer möglichst stabilen Hülle.

Der Durchmesser der resultierenden Mikrokapseln liegt in der Größenordnung von 3—25 $\mu$m.

Beispiel 2

Die organische Phase wird, wie unter Beispiel 1 (b) beschrieben, hergestellt mit der Änderung, daß als Lösungsmittel 25 g Solvesso 200 (Aromatengemisch der Esso AG) eingesetzt werden.

Die Verkapselung und Nachbehandlung wird ebenfalls, wie unter Beispiel 1 (b) beschrieben, durchgeführt mit der Änderung, daß der äußeren Phase 0,7 g Äthylendiamin in 70 g Wasser als Aminkomponente zugegeben werden.

Die Mikrokapseln fallen in einer Größe von 3—25 $\mu$m an.

Beispiel 3

In 25 g Phthalsäure-di-n-butylester werden 0,75 g p-Toluolsulfinat von Michlers Hydrol als farbgebende Komponente unter Rühren und Erwärmen auf 80°C gelöst.

Nach Abkühlen der Lösung werden 5 g des unter Beispiel 1 (a) beschriebenen Oxadiazinons zugegeben und gelöst.

Die resultierende organische Phase wird, wie unter Beispiel 1 (b) beschrieben, verkapselt und nachbehandelt. Als Aminkomponente werden 0,7 g Diäthylentriamin in 70 g Wasser eingesetzt.

Die resultierenden Mikrokapseln haben Durchmesser von 2—20 $\mu$m.

Beispiel 4

(a) Herstellung des wandbildenden Materials

280 g 1,4-Tetramethylen-diisocyanat werden durch Einleiten mit trockenem $CO_2$-Gas gesättigt und unter Rühren und weiterem Einleiten von Kohlendioxid bei 60°C mit 0,7 g Tri-n-butyl-phosphin (0,25%) versetzt. Nach 2 Stunden ist der NCO-Wert auf 53,8% gefallen. Die Reaktionsmischung wird in einem Dünnschichtverdampfer (Heiztemperatur 180°C bei 1 Torr) getrennt, wobei 219 g 1,4-Tetramethylendiisocyanat zurückgewonnen und 54 g eines dickflüssigen Öles erhalten werden. Dieses zeigt die für isocyanatgruppenhaltige Oxadiazintrione charakteristischen Banden im IR-Spektrum (4,4; 5,48; 5,71; 5,83; 6,93; 7,06 $\mu$m), weist einen NCO-Wert von 20,2% auf und ein Molekulargewicht von 375.

(b) Verkapselung

In 25 g einer Mischung aus 70 Teilen Santosol 340 (teilhydriertes Terphenyl der Firma Monsanto) und 30 Teilen Solvesso 200 (Aromatengemisch der Esso AG) werden 1,1 g Kristallviolettlacton bei einer Temperatur von 80°C unter Rühren gelöst.

Nach dem Abkühlen der Lösung werden 5 g des unter 4 (a) beschriebenen Oxadiazinons zugegeben und gelöst. Die Lösung, die die organische Phase darstellt, wird anschließend in 150 g Wasser emulgiert, das 0,75% Mowiol 26—88 (Polyvinylalkohol der Hoechst AG) als Emulgierhilfsmittel enthält. Zum Emulgieren wird eine Kotthoff-Mischsirene verwendet (8900 U/Min., 500 ml Becherglas, Rührzeit ca. 1 Minute). Nach dem Emulgieren wird dem Ansatz eine Lösung von 0,9 g 1-Aminoäthyläthylendiamin-1,2 (Diäthylentriamin) in 50 g Wasser zugesetzt und anschließend die Mischsirene durch einen Laborrührer vom Typ Lenart-Rapid (500 U/min.) ersetzt. Die Nachrührzeit beträgt ca. 1 Std. bei 60°C.

Die Mikrokapseln fallen mit einem Durchmesser von 1—35 $\mu$m an.

Beispiel 5

Die Herstellung einer Mikrokapsel-Dispersion mit ca. 30% Kapselanteil läßt sich mit Hilfe eines Ultraschall-Homogenisators (Typ Minisonic der Fa. Ultrasonics) mit einer Lösung von 4,5 Teilen Kristallviolettlacton und 17 Teilen des unter Beispiel 1 (b) beschreibenen Oxadiazinons in 75 Teilen Santosol 340 (teilhydriertes Terphenyl der Fa. Monsanto und 25 Teilen Solvesso 100 (Esso AG) als organische Phase durchführen.

Zur Emulgierung werden 150 Teile der organischen Phase auf 250 Teil wäßrige Phase eingesetzt. Die wäßrige Phase enthält 0,5% Mowiol 26—88 und 0,1% Tween 80 (Emulgator der Fa. Atlas Chemie) als Emulgierhilfsmittel.

Insgesamt werden die beiden Phasen bzw. die hieraus hergestellte Emulsion 5mal durch den Ultraschall-Homogenisator gegeben. Unmittelbar anschließend wird die resultierende Emulsion in ein Becherglas überführt und unter Rühren mit einem Laborrührer (Typ Lenart-Rapid, 500 U/Min.) zügig mit

der dem NCO-Wert des eingesetzten Oxadiazinons stöchiometrisch entsprechenden Menge Äthylendiamin versetzt (als wäßrige 4%ige Lösung).

Die entstehende Mikrokapseldispersion wird bei Raumtemperatur ca. 1 Std. nachgerührt. Die erhaltenen Mikrokapseln liegen im Bereich von 1—30 $\mu$m Durchmesser.

Beispiel 6

In 25 g Benzoesäureäthylester werden unter Rühren und Erwärmen auf 80°C 0,75 g p-Toluolsulfinat von Michlers Hydrol gelöst.

Nach Erkalten des Ansatzes werden 5 g des unter Beispiel 1 (a) beschriebenen Oxadiazinons zugelöst.

Die resultierende organische Phase wird mit Hilfe einer Kotthoff-Mischsirene bei 8900 U/Min. in 300 g Wasser emulgiert, welches 0,5% Mowiol 56—98 als Emulgierhilfsmittel enthält.

Nach ca. 40 Sek. Mischzeit wird dem Ansatz eine Lösung von 0,7 g Diäthylentriamin in 70 g Wasser zugegeben. Nach ca. 1 Min Emulgierzeit wird die Mischsirene durch einen Laborrührer ersetzt (Typ Lenart-Rapid, 500 U/Min.) und die Mikrokapseldispersion ca. 1 Std. unter Rühren auf 70°C gehalten.

Die Mikrokapseln besitzen Durchmesser von 3—25 $\mu$m.

**Patentansprüche**

1. Reaktionsdurchschreibepapier, das eine Lösung eines Farbbildners in mikroverkapselter Form enthält, wobei der Farbbildner als Lösung in einem hydrophoben organischen Lösungsmittel eingekapselt ist, dadurch gekennzeichnet, dass die Kapselwände aus dem Polyadditionsprodukt eines Diisocyanats der Formel I

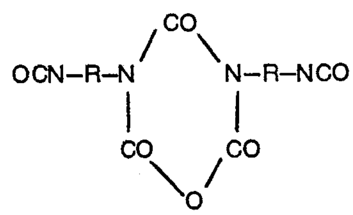

(I)

worin

R einen Alkylenrest mit 2—10 C-Atomen bedeutet, und einem Diamin bestehen.

2. Verwendung von eine Lösung eines Farbbildners in einem hydrophoben organischen Lösungsmittel als Kernmaterial enthaltende Mikrokapseln, deren Wände aus dem Polyadditionsprodukt eines Diisocyanats der Formel I

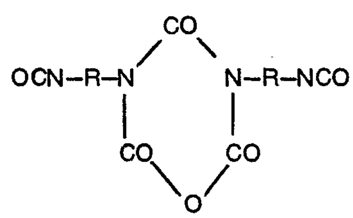

(I)

worin

R einen Alkylenrest mit 2—10 C-Atomen bedeutet, und einem Diamin bestehen, zur Herstellung von Reaktionsdurchschreibepapieren.

**Revendications**

1. Papier carbone à réaction, qui contient une solution d'un chromogène sous la forme microencapsulée, le chromogène étant encapsulé sous forme de solution dans un solvant organique hydrophobe, caractérisé en ce que les parois des capsules sont constituées par le produit de polyaddition d'un diisocyanate de formule I:

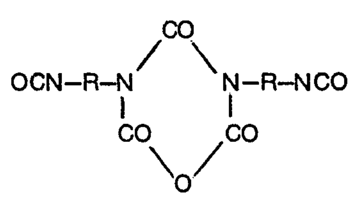

(I)

dans laquelle:

R est un reste alkylène ayant 2—10 atomes de carbone, et d'une diamine.

2. Utilisation de microcapsules contenant comme matière interne une solution d'un chromogène dans un solvant organique hydrophobe, dont les parois sont constituées par le produit de polyaddition d'un diisocyanate de formule I:

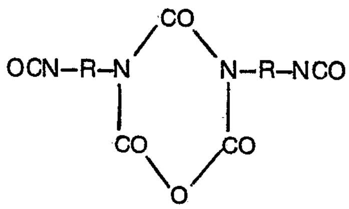

(I)

dans laquelle:

R est un reste alkylène ayant 2—10 atomes de carbone, et d'une diamine, pour la production de papiers carbones à réaction.

**Claims**

1. Pressure-sensitive copying paper, which contains a solution of a dye-precursor agent in micro-encapsulated form, wherein the dye-precursor agent is encapsulated as a solution in a hydrophobic organic solvent, characterised in that the capsule walls consist of the polyaddition product of a diisocyanate of the formula I

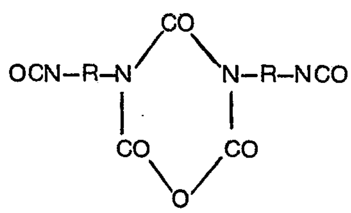

(I)

wherein

R denotes an alkylene radical having from 2 to 10 carbon atoms and a diamine.

2. Use of microcapsules containing as core material a solution of a dye-precursor agent in a hydrophobic organic solvent, the walls of which microcapsules consist of the polyaddition product of a diisocyanate of the formula I

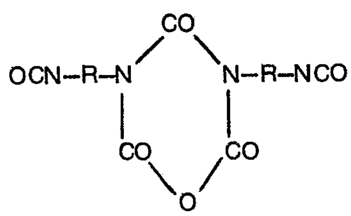

(I)

wherein

R denotes an alkylene radical having from 2 to 10 carbon atoms and a diamine, for the production of pressure-sensitive copying papers.